(19) Europäisches Patentamt
European Patent Office
Office européen des brevets

(11) Publication number: 0 176 616
A1

(12) EUROPEAN PATENT APPLICATION

(21) Application number: 84111837.5

(22) Date of filing: 03.10.84

(51) Int. Cl.[4]: C 07 C 21/02
C 07 C 17/08

(43) Date of publication of application:
09.04.86 Bulletin 86/15

(84) Designated Contracting States:
BE DE FR GB IT NL

(71) Applicant: THE DOW CHEMICAL COMPANY
2030 Dow Center Abbott Road P.O. Box 1967
Midland, MI 48640(US)

(72) Inventor: Klun, Robert T.
4161 E.Baker Road Route 11
Midland Michigan 48640(US)

(72) Inventor: Murchison, Craig B.
606 West Meadowbrook
Midland Michigan 48640(US)

(72) Inventor: Hucul, Dennis A.
5001 Foxcroft
Midland Michigan 48640(US)

(74) Representative: Weickmann, Heinrich, Dipl.-Ing. et al,
Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820
D-8000 München 86(DE)

(54) **Catalytic preparation of 2-halo-1-alkenes from unsaturated hydrocarbons.**

(57) A 2-halo-1-alkene (such as 2-chloro-1-propene) is prepared by contacting together, in the presence of a catalyst (such as alumina), (a) a 1,2-diene (such as propadiene) and/or a 1-alkyne (such as propyne), with (b) a hydrogen halide (such as HCl); in the presence of at least 1,000 ppm water. The process of the invention provides unexpected selectivity for the desired product.

EP 0 176 616 A1

Croydon Printing Company Ltd

0176616

# CATALYTIC PREPARATION OF 2-HALO-1-ALKENES FROM UNSATURATED HYDROCARBONS

The invention relates to an improved process for the preparation of 2-halo-1-alkenes from light hydrocarbon streams.

Griesbaum, US 3,377,389, April 9, 1968, teaches that hydrogen chloride and hydrogen bromide will add to unsaturated compounds in accordance with Markovnikov's Rule and that allene (propadiene) and methylacetylene (propyne) are hydrohalogenated to give 2-halopropene.

Kovachic and L. C. Leitch, "Organic Deuterium Compounds", Can. J. Chem., 39, 363-374 (1961) teach that 2-halo-1-alkenes can be prepared by addition of hydrogen chloride to 1-alkynes. It is taught this addition can be performed in the presence of dibenzoyl peroxide or under ultraviolet illumination.

Arganbright, US 3,142,710, teaches that propyne and propadiene can be selectively halogenated in the presence of propylene (propene). Propylene containing minor amounts of propyne and propadiene together with hydrogen chloride are passed over a catalyst comprising mercuric halide deposited on a porous carrier, particularly one having high surface activity at a temperature in the range of from about 100°C to about 120°C.

Schroeder and Slewka, French Patent 1,505,099 (December 18, 1965), teach that a $C_3$ hydrocarbon stream may be purified by selectively removing propadiene (allene) and propyne (methyl acetylene) by reacting the $C_3$ hydrocarbon stream with hydrogen chloride in the presence of hydrochlorination catalysts, in particular mercuric chloride on a support such as activated aluminum oxide, silica gel or activated carbon. The propadiene and propyne are selectively halogenated to prepare 2-chloropropene which is easily separated from the other components of the $C_3$ light hydrocarbon stream.

Herbertz, "Von Aliphatischen Acetylenkohlenwasserstoffen ausgehende Synthesen", Chem. Ber., 92, 540--50 (1959), teaches two processes for the addition of hydrogen halides to alkynes. One is a mixed phase reaction wherein copper (I)-chloride is the catalyst. In the other, the alkyne is vaporized and mixed with hydrogen chloride and passed over activated charcoal in the presence of mercury at a temperature of 130°C.

A process has now been discovered for selectively halogenating a 1,2-diene and a terminal acetylene with a hydrogen halide in the presence of other

hydrocarbons wherein the yields of the 2-haloalkenes are substantially higher than prior art processes permit. The 2-halo-1-alkenes prepared by this process can be carbonylated and esterified to prepare acrylate esters if desired.

One such acrylate ester is methyl methacrylate which is prepared commercially from acetone which is treated with HCN to give 2-cyano-2-propanol which is then treated with 98 percent sulfuric acid to give a salt of propene-2-amide and sulfuric acid. The salt is then reacted with methanol to prepare methyl methacrylate and $NH_4HSO_4$. This process requires a large purification scheme and the use of sulfuric acid and hydrogen cyanide. It is desirable to prepare methacrylates without the use of sulfuric acid and hydrogen cyanide.

Briefly, the invention is a process for producing a 2-halo-1-alkene by contacting together, in the presence of a catalyst: (a) a 1,2-diene and/or a 1-alkyne; and (b) a hydrogen halide; in the presence of at least 1000 ppm water. The process of the invention unexpectedly provides greater selectivity than prior processes. This increased selectivity was even more unexpected when the inventors of the instant invention discovered the previously unreported fact that the unsaturated hydrocarbon streams used in the analogous prior art processes inherently contain up to about 75 ppm of water.

In this invention, a hydrocarbon stream comprising a 1,2-diene, a 1-alkyne, or mixtures thereof, is reacted with a hydrogen halide to prepare a 2-halo-1-alkene. This can be represented by the following equations:

$$CH_2{=}C{=}CHR + HX \xrightarrow[\text{catalyst}]{H_2O} CH_2{=}\overset{X}{\overset{|}{C}}{-}CH_2R \qquad (I)$$

or

$$CH{\equiv}C{-}CH_2R + HX \xrightarrow[\text{catalyst}]{H_2O} CH_2{=}\overset{X}{\overset{|}{C}}{-}CH_2R \qquad (II)$$

wherein X is chlorine, bromine, iodine or fluorine; and wherein R is hydrogen, alkyl, cycloalkyl, or aryl, and may be substituted or unsubstituted. Such substitution is limited, of course, to those moieties which will not cause substantial chemical or sterical interference with the reaction. Since the prior art reactions employ reactants similar to those used in this invention, suitable substituent moieties will be apparent to those skilled in the art. The 2-halo-1-alkene can be separated from the unreacted hydrocarbons, hydrogen halide and any by-products by conventional means, such as distillation.

The hydrocarbon streams as used herein can contain saturated alkanes, alkenes, dienes and alkynes. A $C_3$ hydrocarbon stream as used herein can contain propane, propylene, propadiene and propynes with minor amounts of $C_2$, $C_4$ and $C_5$ aliphatic compounds. In such streams, the active species are the 1,2-dienes and the 1-alkynes.

The hydrocarbon stream which is halogenated preferably comprises a 1,2-diene of equation I, terminal acetylene of equation II, or mixtures thereof (hereinafter

referred to as active species) wherein R is $C_1$ to $C_{10}$ alkyl or hydrogen. More preferably, R is hydrogen; thus the 1,2-diene is preferably propadiene and the 1-alkyne is preferably propyne. In this more preferred embodiment, any hydrocarbon stream containing propadiene, propyne, or a mixture thereof can be used. A hydrocarbon stream consisting essentially of propadiene and propyne can be used, but it is preferable that the hydrocarbon stream have less than 65 percent of these active compounds, as above this concentration, such a stream is explosive. A hydrocarbon stream of $C_3$ compounds including the active species is a good starting stream.

2-Halopropene is produced where the hydrocarbon stream is a $C_3$ stream containing propadiene, propyne or mixtures thereof.

The hydrogen halide can be hydrogen bromide, hydrogen chloride, hydrogen iodide or hydrogen fluoride. Hydrogen chloride and hydrogen bromide are preferred.

The halogenation of the active species in the hydrocarbon stream can be done in either the liquid phase or the vapor phase. The vapor phase is preferred because in the liquid phase higher concentrations of the active species may be present, increasing the risk of explosion.

Surprisingly, suitable catalysts include carbon, silica alumina, silica gel, silica magnesia, silicalite, aluminosilicates, group IIIA, IIIB, IVA, IVB or V metal oxides or rare earth oxides. The catalysts may be impregnated with mercuric halide or barium bromide as is taught in US 3,142,710, but contrary to the teachings in that patent, such compounds are not necessary to catalyze

this reaction. Preferred catalysts are carbon, silica alumina, silica gel, silica magnesia, silicalite, aluminosilicates, and group IIIA oxides. More preferred are carbon and alumina. The most preferred catalyst is alumina. The catalyst has a significant effect on the selectivity of the insertion of the halogen on the unsaturated active species in the hydrocarbon stream. Those aluminas with high surface area, high acidity and with low silica content demonstrate the best activity.

These catalysts can be regenerated by passing water saturated air through the catalyst for a period of several hours at elevated temperatures. Preferably, the catalyst is regenerated by passing water vapor and an oxygen-containing gas over the catalyst at between about 350°C and 500°C for between 2 to 24 hours.

It has been discovered that the presence of water in the halogenation of the active unsaturated hydrocarbons maintains the selectivity of the catalyst during prolonged use. Furthermore, the presence of the water unexpectedly reduces rate of deactivation of the catalyst. After a short period, four or five hours, the selectivity of the catalyst drops significantly in the absence of water. The use of water during the process maintains the selectivity of the catalyst over the catalyst lifetime.

Only a relatively small amount of water is necessary. That amount of water which is capable of maintaining the selectivity of the catalyst for the insertion of the halide on the 2 carbon of the 1,2-diene or 1-alkyne is sufficient. Preferably, water is present at between 1,000 and 100,000 parts per million by weight,

and more preferably between 3,000 and 30,000 parts per million by weight, based on the weight of the hydrocarbon feed stream. The water can be added by any manner known in the art which provides sufficient water to maintain catalyst selectivity and reduces the rate of catalyst deactivation. The water can be added continuously or intermittently. In fact, if the water level is allowed to drop below that level at which the advantages of this invention are achieved, subsequent addition of water to raise the water level to the levels at which such advantages occur will result in such advantages occurring. In one embodiment, the water can be pumped into the reactor either continuously or intermittently. In another embodiment, water addition may be achieved by saturating the hydrocarbon stream with water vapor prior to contacting it with the hydrogen halide and the catalyst.

The hydrogen halide can be added in any ratio to the active unsaturated compounds in the hydrocarbon stream. A desirable molar ratio is between 0.7 and 1.5 moles of hydrogen halide per mole of active species. The use of excess hydrogen halide may result in the halogenation of compounds in the hydrocarbon stream other than the active unsaturated species and the preparation of 1,2-dihaloalkanes from the active species.

This process can be run over a wide range of temperatures and is desirably run at between -78°C and 400°C. It has been discovered that a preferable temperature range is between 100°C and 300°C. Below 100°C selectivity is reduced and above 300°C little improvement in selectivity is found. Above 400°C there is

considerable degradation of the catalyst. Most preferably the temperature is between 200°C and 300°C as selectivity is at its optimum in this range. In the vapor phase process where the streams of reactants are passed over the catalyst it is desirable to preheat the streams to the reaction temperature prior to passing them over the catalyst.

This step may be run at subatmospheric, atmospheric, or superatmospheric pressure. It is believed that superatmospheric pressures would improve the rate of reaction.

The 2-halo-1-alkenes prepared by the invented process can be isolated by conventional means and carbonylated and esterified to prepare an acrylate ester.

Example 1 - Hydrobromination of a Crude $C_3$ Stream Using Alumina

Through a tube reactor, heated to 215°C at atmospheric pressure and loaded with 15 $cm^3$ of alumina plus inert quartz chips packing, was passed 27.5 ml/min of HBr gas and 90.0 ml/min of a crude $C_3$ stream saturated with water vapor (10,000 ppm). The molar composition of the $C_3$ stream was 16.8 percent propadiene, 22.8 percent propyne, 52.4 percent propylene, 6.5 percent propane and 1.5 percent mixed $C_4$ hydrocarbons. Approximately 65 percent total propyne and propadiene conversion was achieved with about 95 percent selectivity to 2-bromopropene.

Example 2 - Hydrochlorination of a Crude $C_3$ Stream Using Alumina

Through a tube reactor, heated to 290°C at atmospheric pressure and loaded with 50 $cm^3$ of alumina plus inert quartz chips packing, was passed 73.0 ml/min of HCl gas and 150 ml/min of a crude $C_3$ stream saturated with water vapor (10,000 ppm). The molar composition of the $C_3$ stream was 24.0 percent propadiene, 27.2 percent propyne, 18.8 percent propylene, 23.4 percent propane and 6.6 percent mixed $C_4$ hydrocarbons. Approximately 73 percent total propyne and propadiene conversion was achieved with approximately 98 percent selectivity to 2-chloropropene.

Examples 3-10

Examples 3-10 were run in a manner similar to Examples 1 and 2. The results are compiled in Table I. In Table I, actives conversion refers to the percentage of propyne and propadiene which was halogenated and selectivity refers to the percentage of the 2-halopropene in such halogenated compounds.

Table I demonstrates that in the process of the invention, alumina is preferred over carbon. Further, Examples 1-3 demonstrate that the presence of water increases the selectivity of the reaction for 2-halopropene.

Table II shows the composition of the $C_3$ stream of Examples 1 to 10.

TABLE I

| Example No. | Hydrogen Halide | Catalyst | Temp °C | Water Vapor | HX flow rate | $C_3$ Stream flow rate ml/min | Conversion % | Selectivity % |
|---|---|---|---|---|---|---|---|---|
| 1 | HBr | Alumina | 215 | yes | 27.5 | 90.0 | 65 | 95 |
| 2 | HCl | Alumina | 290 | yes | 73.0 | 150.0 | 73 | 98 |
| 3* | HBr | Alumina | 220 | no | 19.0 | 106.0 | 65 | 90 |
| 4* | HBr | Carbon impregnated with 28% BaBr and 0.7% HgBr | 215 | no | 35.5 | 120.0 | 70 | 84 |
| 5* | HBr | Zirconia | 265 | no | 27.5 | 90.0 | 42 | 73 |
| 6* | HBr | Silica Alumina | 240 | no | 36.0 | 100.0 | 30 | 53 |
| 7* | HBr | Silicalite | 222 | no | 36.0 | 100.0 | 15 | 28 |
| 8* | HBr | Titania | 275 | no | 31.0 | 103.0 | 41 | 79 |
| 9* | HBr | Magnesia | 212 | no | 28.0 | - | 5 | 38 |
| 10 | HBr | Carbon | 265 | yes | 25.0 | 96.0 | 60 | 83 |

*Not an example of the invention.

TABLE II

Composition of $C_3$ Streams
Examples 1-10

| Example | Propadiene | Propyne | Propylene | Propane | $C_4$ Hydrocarbons |
|---|---|---|---|---|---|
| 1 and 5-9 | 16.8 | 22.8 | 52.4 | 6.5 | 1.5 |
| 2 | 24.0 | 27.2 | 18.8 | 23.4 | 6.6 |
| 3 | 11.7 | 14.4 | 46.6 | 27.0 | 0.4 |
| 4 and 10 | 16.4 | 20.6 | 54.6 | 6.8 | 1.5 |

Example 11 - Effect of Temperature on the Selectivity of the Halogenation of a $C_3$ Hydrocarbon Stream to 2-Chloropropene

A $C_3$ hydrocarbon stream containing propadiene and propyne and a stream of HCl were passed over an alumina catalyst for 98.5 hours. The $C_3$ stream was bubbled through water (to provide 10,000 ppm water) prior to passing over the catalyst. The temperature was gradually increased from 155.1°C to 307.8°C over the 98.5-hour period. Periodic samples were taken and analyzed. Table III shows the effect of temperature on the selectivity for 2-chloropropene of the chlorinated methyl acetylene and propadiene.

TABLE III

| Time (hours) | Temp. °C | Selectivity (%) |
|---|---|---|
| 6.00 | 155.6 | 50.8 |
| 9.25 | 177.6 | 71.5 |
| 13.75 | 198.0 | 84.8 |
| 17.50 | 216.0 | 92.1 |
| 31.25 | 231.0 | 93.9 |
| 52.30 | 231.0 | 91.1 |
| 63.00 | 251.7 | 98.2 |
| 77.45 | 271.1 | 98.5 |
| 86.00 | 331.1 | 98.8 |
| 92.25 | 307.8 | 97.5 |

This demonstrates that selectivity improves with an increase in temperature and that above 300°C there is little improvement in selectivity.

Example 12 - Effect of Water Vapor on the Selectivity of the Halogenation of a $C_3$ Hydrocarbon Stream to 2-Bromopropene

A $C_3$ hydrocarbon stream containing propadiene and propyne and a stream of HBr were passed over an alumina catalyst for 20.3 hours at a temperature ranging from 250°C to 303°C. The hydrocarbon stream was bubbled through water (to provide 10,000 ppm water) prior to passing it over the catalyst. Table IV shows the selectivity over time.

TABLE IV

| Time (hours) | Selectivity for 2-Bromopropene (%) |
|---|---|
| 2.0 | 100.0 |
| 3.0 | 100.0 |
| 4.5 | 99.2 |
| 5.5 | 95.9 |
| 6.5 | 93.0 |
| 7.5 | 91.5 |
| 8.5 | 93.9 |
| 9.5 | 92.0 |
| 10.5 | 91.3 |
| 14.0 | 94.1 |
| 15.0 | 93.9 |
| 16.3 | 95.1 |
| 18.3 | 95.2 |
| 19.3 | 94.1 |
| 20.3 | 93.0 |

A $C_3$ hydrocarbon stream containing propadiene and propyne and a stream of HBr were passed over an alumina catalyst for 23.5 hours at a temperature ranging from 200°C to 220°C. The $C_3$ stream was not bubbled through water. The selectivity over time is shown in Table V.

TABLE V

| Time (hours) | Selectivity for 2-Bromopropene (%) |
|---|---|
| 2.1 | 100.0 |
| 5.5 | 97.6 |
| 6.6 | 93.5 |
| 7.5 | 90.1 |
| 8.5 | 86.0 |
| 9.4 | 83.7 |
| 10.4 | 80.9 |
| 14.3 | 73.0 |
| 15.4 | 71.2 |
| 17.6 | 68.5 |
| 18.5 | 67.9 |
| 19.4 | 66.4 |
| 20.4 | 64.7 |

The above data demonstrates that the presence of 10,000 ppm of water kept the selectivity of the reaction for 2-bromopropene above 90 percent, whereas in the absence of the water, the selectivity of the reaction steadily decreased to 65 percent over the 20.3-hour period. This demonstrates that the presence of water has a significant effect in maintaining the selectivity for 2-bromopropene above 90 percent for a longer period of time.

0176616

Example 13

Through a tube reactor loaded with 10 $cm^3$ of γ-alumina catalysts and heated to 290°C, is passed HCl at a flow rate of 55 $cm^3$/min, and a $C_3$ stream at a flow rate of 200 $cm^3$/min. The pressure is 5 psig (34 kPa, gauge). The $C_3$ stream comprises 18.19 percent allene (propadiene), 22.23 percent methyl acetylene (propyne), 1.39 percent isobutylene, 7.61 percent propane, 45.76 percent propylene, and 4.13 percent N-butane. Water is added to provide a water concentration of 3,900 parts per million. The process is repeated at a water concentration of 75 parts per million and 12,000 parts per million. The rate of deactivation of the catalyst is calculated at each concentration. The results are compiled in Table VI.

TABLE VI

| Water Concentration ppm | Deactivation Rate Percent per hour |
|---|---|
| 75 | 1.8 |
| 3,900 | 0.36 |
| 12,000 | 0.07 |

0176616

1. A process for preparing a 2-halo-1-alkene comprising contacting together in the presence of a catalyst,

(a) a 1,2-diene of the formula $CH_2=C=CH-R$, a 1-alkyne of the formula $CH\equiv C-CH_2-R$, or a mixture thereof, wherein each R independently represents a substituted or unsubstituted moiety comprising hydrogen, alkyl, cycloalkyl, or aryl; and

(b) a hydrogen halide;

characterized in that there is present during the reaction, at least 1000 ppm of water.

2. The process of Claim 1 wherein the catalyst comprises carbon, silica alumina, aluminosilicates, silica gel, silica, silica magnesia, silicalite, group IIIA, IIIB, IVA, IVB or V metal oxides or rare earth oxides.

3. The process of Claim 2 wherein the catalyst is carbon or alumina.

0176616

4. The process of Claim 1, 2 or 3 wherein the reaction is conducted in the substantial absence of mercuric halides, copper halides, aluminum halides, iron halides, zinc halides, bismuth halides, nickel halides or calcium halides.

5. The process of Claim 1, 2 or 3 wherein R is H or $C_1$ to $C_{10}$ alkyl.

6. The process of Claim 5 wherein the 1,2--diene is propadiene, the 1-alkyne is propyne and the 2-halo-1-alkene is 2-halopropene.

7. The process of Claim 1 wherein the hydrocarbon stream is contacted with the hydrogen halide in the vapor phase.

8. The process of Claim 1 wherein the temperature is between 100°C and 400°C.

9. The process of Claim 1 wherein the hydrocarbon stream is saturated with water prior to contacting the stream with hydrogen halide and the catalyst.

10. The process of Claim 1 wherein the water concentration is between 1,000 and 100,000 parts per million by weight.

European Patent Office

# EUROPEAN SEARCH REPORT

0176616

Application number

EP 84 11 1837

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| A | US-A-2 446 124 (T.BOYD)<br>--- | | C 07 C 21/02<br>C 07 C 17/08 |
| A | GB-A-1 141 108 (INTERNATIONAL FLAVORS & FRAGRANCES)<br>* Page 2, lines 54-57 *<br>--- | | |
| A,D | US-A-3 377 389 (K.GRIESBAUM)<br>--- | | |
| A,D | US-A-3 142 710 (R.P.ARGANBRIGHT)<br>----- | | |
| | | | TECHNICAL FIELDS SEARCHED (Int Cl 4) |
| | | | C 07 C 17/00 |

The present search report has been drawn up for all claims

| Place of search | Date of completion of the search | Examiner |
|---|---|---|
| THE HAGUE | 20-05-1985 | VAN GEYT J.J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82